# DEMANDE DE BREVET EUROPEEN

(11) **EP 2 923 729 A1**
(43) Date de publication de la demande: **30.09.2015**
(21) Numéro de dépôt: 15157720.2
(22) Date de dépôt: 05.03.2015
(51) Int. Cl.: A61N 5/06

(54) **MODULE PORTATIF DE TRAITEMENT PAR PHOTOTHÉRAPIE**

(30) Priorité: 24.03.2014 FR 1452441
(71) Demandeur: Lucibel SA, 92500 Rueil Malmaison (FR)
(72) Inventeur: Houot, Jean-Laurent, 38110 Saint-Clair-de-la-Tour (FR)
(74) Mandataire: Martin, Marie-Aude

(57) **Abrégé**

Ce module portatif (14) comprend un boîtier (24) propre à être saisi par un utilisateur. Ce boîtier (24) est muni d'une fenêtre (26) d'émission d'un flux lumineux. Le module (14) comprend en outre une source (32) de génération d'un flux lumineux à travers la fenêtre (26) pour éclairer la zone. Il comprend des moyens de liaison du module (14) aptes à coopérer avec des moyens de liaison complémentaires (38B) d'un autre module. Les moyens de liaison sont agencés pour permettre une liaison mécanique libérable des deux modules entre eux et sont électriquement conducteurs pour autoriser le passage d'un courant électrique entre les deux modules.

## Description

La présente invention concerne un équipement de traitement d'une région externe du corps d'un être vivant par photothérapie. Elle s'applique plus particulièrement mais non exclusivement au traitement de la peau du corps ou du visage d'un patient humain.

Le traitement consiste en l'application d'un rayonnement lumineux présentant un spectre d'émission prédéfini directement sur la région du corps à traiter. Un tel traitement peut être appliqué également en combinaison avec un produit photo-sensibilisant appliqué sur la région à traiter quelques heures avant son exposition au rayonnement lumineux pour augmenter l'efficacité du traitement. On parle alors dans ce cas de photothérapie dynamique (connue également sous l'acronyme anglais PDT « Photo Dynamic Therapy »).

Le rayonnement est de préférence sensiblement monochromatique ou présente un spectre d'émission compris dans une bande étroite de longueurs d'onde. Généralement, le spectre d'émission comprend une longueur d'onde maximale située dans la lumière visible rouge ou encore dans la lumière visible bleue, ces longueurs d'onde ayant démontré une efficacité particulière pour le traitement de certaines affections de la peau.

Il a été ainsi observé au cours de nombreuses études scientifiques, qu'un traitement par photothérapie dans une plage de longueurs d'onde prédéfinie, notamment dans la lumière rouge ou bleue, permet d'agir de façon bénéfique sur de nombreux problèmes cutanés, tels que les cicatrices, les vergetures, l'acné, les rides et ridules, mais également certains cancers cutanés.

Plus particulièrement, lors de l'application du rayonnement sur la peau, notamment dans la lumière rouge et plus particulièrement autour de la longueur d'onde 630 nm, les cellules de la région irradiées vont réagir selon un principe similaire à celui de la photosynthèse des végétaux. La lumière va stimuler les cellules, et plus spécifiquement certaines structures cellulaires de la cellule, telles que les mitochondries.

De façon connue en soi, les mitochondries jouent un rôle particulièrement important dans le processus de respiration cellulaire ainsi que dans le processus de transformation des nutriments en un vecteur énergétique désigné couramment par ATP (acronyme pour Adénosine Tri Phosphate). Dès lors, la stimulation des mitochondries par la lumière rouge contribue à une régénération des cellules grâce à l'amélioration du processus de respiration et d'alimentation des cellules. Une telle régénération cellulaire va se manifester par exemple pour les cellules du visage par une augmentation de la production de collagène et d'élastine et par conséquent une diminution des rides et ridules.

Il est déjà connu dans l'état de la technique, notamment du document US 6 626 932, une installation de traitement d'un patient par photothérapie. Ce document décrit une installation particulièrement complexe et encombrante. L'installation permet de traiter des surfaces larges du corps de l'utilisateur et comprend à cet effet un habitacle dimensionné pour permettre au patient de s'allonger ou de se tenir debout ou encore assis. A cet effet, l'installation peut comporter une couchette ou un siège. L'installation comprend en outre une source lumineuse formée par une série de tubes fluorescents de grandes dimensions.

Or, un tel appareil est particulièrement complexe et encombrant. Il est également complexe à fabriquer. Par ailleurs, du fait que le patient est situé à distance des tubes de lumière, ces derniers doivent fournir un rayonnement puissant ce qui les rend particulièrement consommateurs d'énergie. En outre, cette installation n'est pas adaptée pour traiter des petites surfaces de peau, comme cela est nécessaire par exemple pour traiter une cicatrice.

Par conséquent, il existe un besoin d'un équipement de photothérapie qui puisse s'adapter à la dimension de la zone à traiter de l'utilisateur qu'elle soit large ou au contraire étroite et qui ne présente pas les inconvénients précités de l'installation de l'art antérieur, à savoir son encombrement et sa complexité. En outre, l'équipement doit avoir une fabrication peu coûteuse et avoir une consommation énergétique réduite.

A cet effet, l'invention a pour objet un module portatif de traitement d'une zone externe d'un utilisateur par photothérapie du type comprenant un boîtier propre à être saisi par un utilisateur muni d'une fenêtre d'émission d'un flux lumineux et une source de génération d'un flux lumineux à travers la fenêtre pour éclairer la zone, **caractérisé en ce qu**'il comprend des moyens de liaison du module aptes à coopérer avec des moyens de liaison complémentaires d'un autre module, les moyens de liaison étant agencés pour permettre une liaison mécanique libérable des deux modules entre eux et étant électriquement conducteurs pour autoriser le passage d'un courant électrique entre les deux modules.

L'invention permet ainsi d'obtenir d'une façon générale un équipement de traitement par photothérapie modulaire par assemblage d'une pluralité de modules. Le module fait partie d'une pluralité de modules. La configuration modulaire permet ainsi d'adapter la dimension de la surface à traiter en fonction du besoin thérapeutique ou cosmétique. Les modules peuvent être réunis et séparés facilement grâce à leurs moyens de liaison libérables complémentaires. Par ailleurs, comme la liaison est électriquement conductrice, la mise en oeuvre de l'équipement est très simple : aucun branchement électrique additionnel n'est requis lors de l'assemblage des modules entre eux.

Ainsi, du fait que la surface à traiter est en quelque sorte échantillonnée par les modules, il est possible de couvrir des surfaces plus ou moins grandes sans recourir à des installations complexes et encombrantes.

Par liaison mécanique, au sens de l'invention, on entendra la relation des modules entre eux liés par un ou des contacts physiques ou directs qui les rendent totalement ou partiellement solidaires.

Le module de traitement selon l'invention peut comporter l'une ou plusieurs des caractéristiques suivantes.

De préférence, les moyens de liaison et les moyens de liaison complémentaires sont de type à aimantation pour former une liaison magnétique amovible entre les deux modules.

La liaison magnétique permet d'assembler rapidement les modules ensemble et de les détacher aisément. Cette liaison permet également un passage du courant électrique entre les modules lorsque les moyens sont réalisés dans un matériau électriquement conducteur et magnétique.

De préférence, les moyens de liaison et les moyens de liaison complémentaires sont magnétiques de polarité opposée.

Ainsi, du fait que les polarités sont opposées, les moyens de liaison et les moyens de liaison complémentaires exercent l'un sur l'autre une force d'attraction magnétique.

De préférence, la liaison mécanique libérable entre les deux modules est une liaison latérale comprenant au moins un degré de liberté.

Dans un mode de réalisation préféré de l'invention, les moyens de liaison définissent un axe, la liaison mécanique entre les deux modules forme un pivot le long de cet axe.

Du fait qu'il y a une mobilité des modules entre eux, l'ensemble modulaire peut avantageusement épouser au plus près la forme de la surface à traiter. Ceci offre une flexibilité à l'équipement.

De préférence, les moyens de liaison et les moyens de liaison complémentaires comprennent chacun au moins un élément de liaison magnétique dit « individuel » et l'un des moyens de liaison et des moyens de liaison complémentaires comprennent un élément de liaison magnétique dit « mutuel » de forme générale sphérique, cet élément mutuel sphérique étant entièrement séparable des deux modules.

Ceci permet de former une liaison articulée de façon simple. Une telle liaison présente un axe principal d'aimantation permettant une liaison relativement forte selon cet axe mais qui peut être rapidement rompue par un effort de cisaillement selon une direction perpendiculaire à l'axe principal d'aimantation.

De préférence, la liaison mécanique des moyens de liaison et des moyens de liaison complémentaires est de type sphère-plan, sphère-sphère ou sphère-creux.

De préférence, le boîtier comprend des premiers et seconds moyens de liaison aptes à coopérer respectivement avec des premiers et deuxièmes moyens complémentaires respectivement d'un premier autre module et d'un deuxième autre module, les premiers et seconds moyens étant agencés sur deux côtés opposés du boîtier pour le raccordement latéral du module avec les premier et deuxième autres modules.

De préférence, les premiers moyens de liaison et les seconds moyens de liaison du module sont complémentaires l'un de l'autre et sont magnétiques de polarité opposée.

Ceci permet d'assurer la complémentarité de deux modules entre eux de la pluralité de modules. Lorsque les modules identiques entre eux sont mis à proximité les uns des autres, par définition, les premiers moyens de liaison de l'un des modules sont complémentaires des seconds moyens de liaison de l'autre des modules.

De préférence, les moyens de liaison comprenant une paire d'éléments de liaison et les moyens de liaison complémentaires comprenant une paire d'éléments de liaison complémentaires, les éléments de liaison de chaque paire sont magnétiques de polarité opposée afin de définir un unique sens de montage des deux modules entre eux.

Ceci permet d'éviter de créer un court-circuit lors du raccordement de deux modules entre eux. Il s'agit en fait d'un détrompeur.

De préférence, la source de génération lumineuse comprend une pluralité d'unités d'éclairages, chaque unité d'éclairage comprenant au moins une puce DEL.

De préférence, les unités d'éclairage d'une même source sont raccordées en série les uns aux autres et les sources de deux modules sont raccordées en parallèle.

Avantageusement, du fait que les unités d'éclairage sont en série, seulement deux bornes d'alimentation de la source de lumière sont requises.

De préférence, la source lumineuse a un spectre d'émission présentant une longueur d'onde d'émission maximale comprise dans la plage de longueurs d'ondes choisie parmi les plages 400 nm à 440 nm, 610 nm à 650 nm et 810 nm à 850 nm.

De préférence, le module comprend un organe de sécurité apte à éteindre la source lumineuse en cas d'absence de contact d'au moins une partie fenêtre contre un objet extérieur, par exemple l'utilisateur.

Ceci permet d'éviter tout risque d'exposition accidentelle à un rayonnement. En effet, lorsque la partie de la fenêtre n'est pas en contact avec un objet, la source lumineuse est instantanément éteinte.

L'invention a encore pour objet un module d'alimentation électrique d'un module de traitement selon l'invention du type comprenant un boîtier propre à être saisi par un utilisateur, une source d'alimentation électrique raccordée électriquement à des bornes électriques accessibles de l'extérieur du boîtier, et les bornes électriques sont formées par des moyens de liaison complémentaires des moyens de liaison du module de traitement, les moyens de liaison complémentaires étant agencés pour permettre une liaison mécanique articulée libérable des deux modules entre eux.

Le module d'alimentation électrique peut comprendre encore la caractéristique selon laquelle la source d'alimentation électrique étant rechargeable, le module comprend une prise électrique pour le rechargement de la source d'alimentation électrique.

L'équipement est par conséquent autonome puisqu'une fois rechargé, il n'est plus nécessaire de rester à proximité du réseau électrique.

Le module d'alimentation peut comprendre encore la caractéristique selon laquelle comprenant des premier et deuxième moyens de liaison identiques aux premiers et seconds moyens de liaison du module de traitement selon l'invention.

L'invention a encore pour objet un équipement de traitement d'un utilisateur par photothérapie **caractérisé en ce qu**'il comprend une pluralité de modules selon l'invention, comprenant un module d'alimentation et au moins deux modules de traitement et un support apte à s'étendre autour d'une partie du corps de l'utilisateur et sur lequel les modules sont disposés côte à côte.

L'invention a enfin pour objet un équipement de traitement d'un utilisateur par photothérapie, **caractérisé en ce qu**'il comprend un module d'alimentation électrique et un module de traitement selon l'invention **et en ce qu**'il comprend une structure d'interconnexion libérable des deux modules entre eux pour former un ensemble solidaire par superposition des deux modules l'un sur l'autre, cette structure étant apte à relier électriquement les moyens de liaison et les moyens de liaison complémentaires des deux modules pour autoriser le passage d'un courant électrique.

Dans un mode de réalisation préféré de l'invention, la structure comprend un corps muni d'une cloison délimitant des premier et deuxième espaces de réception respectivement du module d'alimentation et du module de traitement et des éléments de contact électrique disposés selon une direction sensiblement transverse aptes à coopérer avec les moyens de liaison des modules.

D'autres caractéristiques et avantages de l'invention apparaîtront à la lumière de la description qui suit, faite en référence aux dessins annexés dans lesquels :
- la figure 1 représente une vue en perspective d'un équipement de photothérapie selon un premier mode de réalisation de l'invention;
- la figure 2 représente une vue partielle de face d'une pluralité de modules de photothérapie de l'équipement de la figure 1 ;
- la figure 3 représente une vue de dessus d'un module de traitement de la pluralité de modules représentée sur la figure 2 ;
- la figure 4 représente une vue en perspective et en coupe selon la ligne 4-4 de la figure 3 ;
- la figure 5 représente une vue éclatée du module de traitement de la figure 3 ;
- la figure 6 représente une vue éclatée d'un module d'alimentation électrique de la pluralité de modules représentée sur la figure 2 ;
- la figure 7 représente une vue en perspective d'une partie intérieure d'un boîtier du module de la figure 5 ou de la figure 6 ;
- les figures 8A et 8B représentent des vues schématiques de détail de moyens de liaisons libérables de deux modules de la figure 2 ;
- la figure 9 représente une vue de côté d'un équipement de photothérapie selon un deuxième mode de réalisation ;
- la figure 10 représente une vue éclatée en perspective d'une partie de l'équipement de photothérapie de la figure 9 ;
- la figure 11 représente une vue en perspective de dessous de l'équipement de la figure 9 ;
- la figure 12 illustre un exemple d'application de l'équipement selon le premier mode de réalisation.

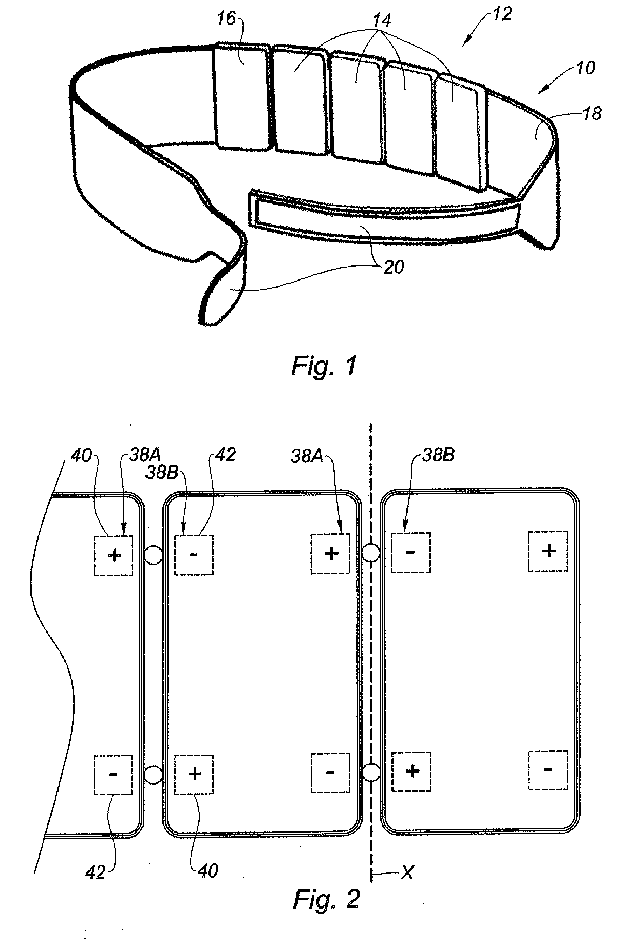

On a représenté de façon schématique sur **la** **figure 1** un équipement de traitement par photothérapie selon un premier mode de réalisation de l'invention. Cet équipement est désigné par la référence générale 10.

Cet équipement 10 comprend une pluralité 12 de modules portatifs selon l'invention. La pluralité 12 de modules comprend au moins un module 14 de traitement d'une zone externe d'un utilisateur par photothérapie, dit module lumineux, et au moins un module 16 d'alimentation électrique de ce module lumineux 14. Dans l'exemple illustré par la **figure 1****,** la pluralité 12 de modules comprend quatre modules lumineux 14 et un module d'alimentation électrique 16 des quatre modules lumineux 14.

L'équipement 10 comprend également dans l'exemple décrit une bande 18 formant support des modules 14, 16. La bande 18 comprend des moyens 20 d'adaptation de la dimension de la bande 18 à la dimension de la partie du corps de l'utilisateur. Les moyens d'adaptation 20 comprennent par exemple des moyens de fermeture choisis parmi des moyens de fermeture du type auto-agrippant, des moyens de fermeture à pression et des moyens de fermeture à boucle/crochet. Dans le cas présent, les moyens de fermeture sont de type auto-agrippant.

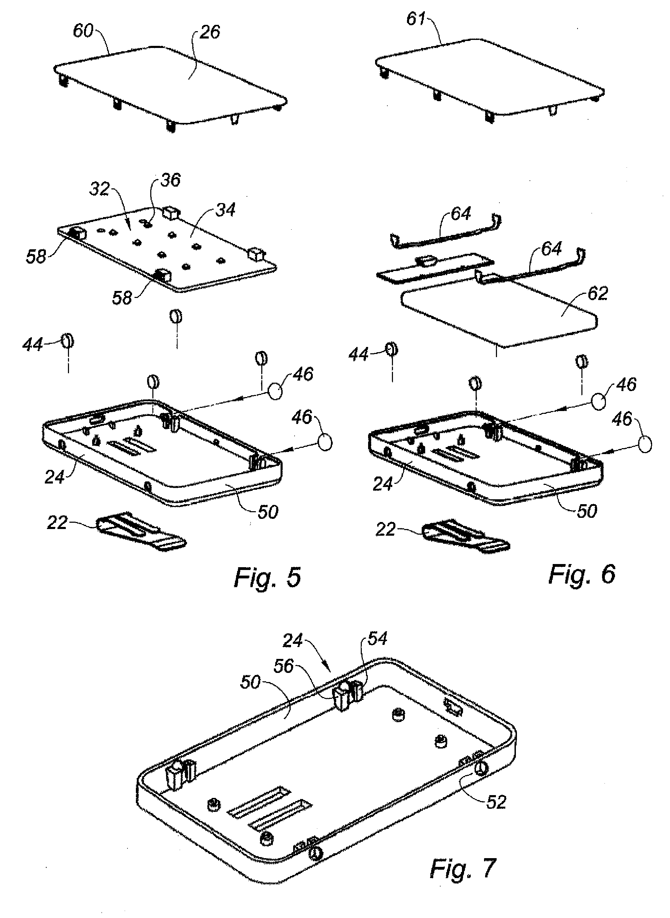

Par ailleurs, de préférence, les modules 14, 16 comprennent des moyens 22 de fixation à la bande 18 de support. Ces moyens de fixation 22 comprennent par exemple des éléments forment pince ou « clip ». Un tel élément 22 est illustré par exemple sur **les** **figures 5 et 6****.**

On va maintenant décrire plus en détail en référence aux **figures 2 à 5****,** un mode de réalisation préféré d'un module lumineux 14 selon l'invention. On ne décrira par la suite qu'un seul module lumineux car les quatre modules lumineux sont identiques entre eux.

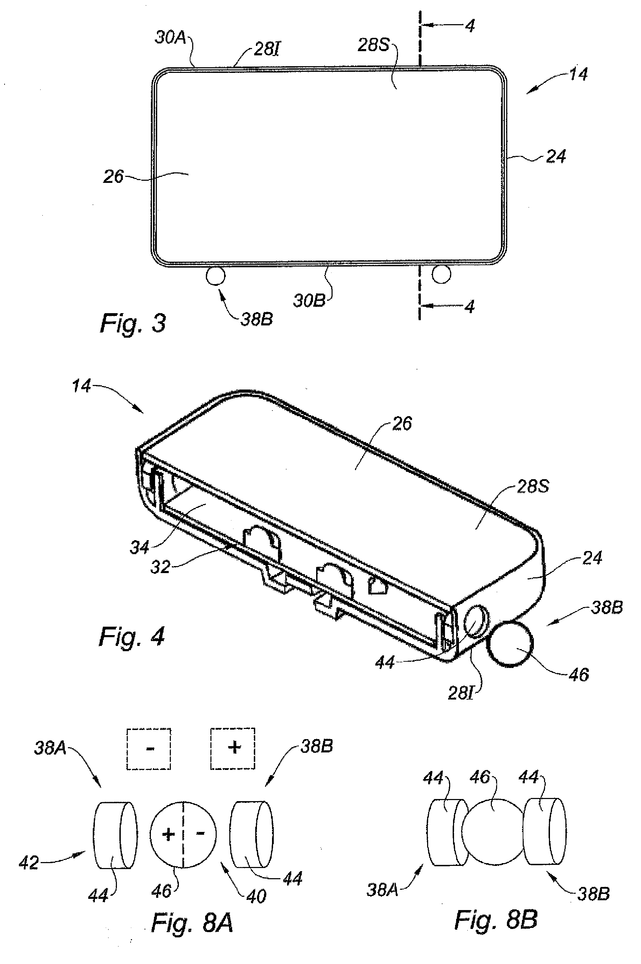

Selon l'invention, le module 14 comprend un boîtier 24 propre à être saisi par l'utilisateur. Comme cela est illustré sur **la** **figure 3****,** le boîtier 24 du module lumineux 14 a une forme générale parallélépipédique délimitant une face supérieure 28S (ou avant) et une face inférieure 28I (ou arrière) et deux côtés opposés 30A, 30B. Dans l'exemple illustré, la face inférieure 28I porte les moyens de fixation 22 du module 14 à la ceinture 18. Ce boîtier 24 est muni d'une fenêtre 26 d'émission d'un flux lumineux. Dans l'exemple décrit, la fenêtre 26 est réalisée dans un matériau translucide, par exemple un matériau plastique tel que du polycarbonate. De préférence, la fenêtre 26 est formée sur une des faces du boîtier 24, par exemple sur la face avant 28S.

En outre, le module lumineux 14 comprend une source 32 de génération d'un flux lumineux à travers la fenêtre 26. Comme cela est illustré par **les** **figures 4** **et** **5****,** la source de lumière 32 est portée par un support 34. Par exemple, ce support 34 forme un support pour un circuit imprimé comprenant notamment un circuit d'alimentation électrique de la source 32.

De préférence et comme cela est illustré sur **la** **figure 5****,** la source de lumière 32 comprend une pluralité d'unités 36 de génération d'un flux lumineux. Chaque unité 36 comprend de préférence un composant électronique de type à diode électroluminescente (désigné ci-après par l'acronyme DEL). Par exemple, le composant électronique comprend de façon classique un boîtier de composant, une embase formant support et au moins une puce semiconductrice DEL portée par l'embase et des pattes de connexion électrique de la puce à des moyens externes tels qu'au circuit électrique d'alimentation de la source de lumière 32. Ce composant peut comporter en outre un système optique permettant d'extraire le flux lumineux produit par la puce semi-conductrice. Le composant est par exemple de type composant monté en surface. Ainsi, dans l'exemple illustré sur la **figure 5****,** les composants électroniques DEL sont montés en surface sur le support 34 de circuit imprimé.

De préférence, la source lumineuse 32 a un spectre d'émission présentant une longueur d'onde d'émission maximale comprise dans la plage de longueurs d'onde choisies parmi les plages 400 nm à 440 nm, 610 nm à 650 nm et 810 nm à 850 nm.

Par ailleurs, le module lumineux 14 comprend en outre de préférence des moyens de pilotage (non représentés) de ces unités lumineuses 36 selon un programme de traitement de photothérapie prédéfini. Ce programme de traitement peut par exemple être mémorisé par les moyens de pilotage.

Le programme de traitement comprend par exemple des instructions de code pour la réalisation d'étapes choisies parmi une étape d'allumage de la source 32, une étape d'extinction de la source 32, une étape de définition d'un mode d'allumage pulsé ou continu de la source 32, une étape de définition d'une durée de période d'allumage de la source 32.

Par exemple, les moyens de pilotage des unités 36 comprennent un microcontrôleur porté par le support 34, apte à mémoriser le programme de pilotage.

De préférence, les unités 36 de la source 32 de lumière sont raccordées électriquement en série entre elles. Ceci permet avantageusement de limiter l'intensité du courant circulant dans le circuit d'alimentation électrique de la source de lumière 32 et ne requérir que seulement deux bornes pour l'alimentation de la source 32.

Plus particulièrement, selon l'invention, le module lumineux 14 comprend des moyens 38A de liaison du module 14 aptes à coopérer avec des moyens de liaison 38B complémentaires d'un autre module 14 ou 16. De façon plus précise et conformément à l'invention, ces moyens de liaison 38A, 38B sont agencés pour permettre une liaison mécanique articulée libérable de deux modules 14 entre eux. Par ailleurs, ces moyens de liaison 38A, 38B sont électriquement conducteurs pour autoriser le passage d'un courant électrique entre les deux modules 14.

De préférence, les moyens de liaison 38A et les moyens de liaison complémentaires 38B sont de type à aimantation pour former une liaison magnétique amovible entre les deux modules de la pluralité de modules.

De préférence, chaque module 14 comprend des premiers 38A et seconds 38B moyens de liaison aptes à coopérer respectivement avec des premier 38B et deuxième 38A moyens de liaison complémentaires respectivement d'un premier autre module 14 et d'un deuxième autre module 14. Dans l'exemple décrit, les premiers 38A et deuxièmes 38B moyens de liaison sont agencés sur deux côtés opposés 30A, 30B du boîtier 24 pour le raccordement latéral du module 14 avec les deux autres modules, comme cela est illustré par **la** **figure 2****.**

De préférence, les premiers 38A et seconds 38B moyens de liaison du module 14 sont complémentaires l'un de l'autre et sont magnétiques de polarité opposée. De ce fait, lorsque deux modules lumineux 14 sont présentés à proximité l'un de l'autre, les premiers moyens de liaison 38A du premier module 14 sont aptes à coopérer avec les seconds moyens de liaison 38B du deuxième module 14 par attraction magnétique puisque par définition, les premiers moyens de liaison 38A sont complémentaires des seconds moyens de liaison 38B d'un même module.

Comme illustré sur **cette** **figure 2****,** les premiers moyens de liaison 38A comprennent une paire d'éléments de liaison 40 et 42 et les moyens de liaison 38B comprennent une paire d'éléments de liaison complémentaires 40, 42. Les éléments de liaison 40 et 42 de chaque paire 38A, 38B sont magnétiques de polarité opposée afin de définir un unique sens de montage des deux modules entre eux.

Toutefois, en variante, les éléments de liaison de chaque paire 38A, 38B peuvent être de polarité identique pour une même paire et ce sont les deux paires 38A, 38B des premiers moyens et des seconds moyens de liaison de chaque module qui ont des polarités opposées.

Bien entendu, en variante, seulement l'un des moyens de liaison 38A, 38B peut être de type aimanté, tandis que l'autre des moyens de liaison complémentaires 38B, 38A peut être de type aimantable, c'est-à-dire être réalisé dans un matériau métallique apte à créer une liaison magnétique avec un aimant.

De préférence, les moyens de liaison 38A et les moyens de liaison complémentaires 38B comprennent au moins un élément de liaison magnétique dit « individuel » 44. On a ainsi représenté en détail sur les figures **8A, 8B****,** deux éléments de liaison complémentaires 40, 42 respectivement d'une paire de moyens de liaison 38A et d'une paire de moyens de liaison complémentaires 38B.

Ainsi, de préférence, l'élément de liaison magnétique 44 est formé par un premier aimant comprenant un pôle mâle et un pôle femelle. Dans l'exemple décrit, ce premier aimant 44 a une forme générale de pastille.

On entend par pastille au sens de l'invention, une pièce en trois dimensions dont la largeur et la longueur sont du même ordre de grandeur, cet ordre de grandeur étant bien supérieur à celui de la hauteur. Cette pastille peut ainsi présenter une face supérieure ou inférieure plate, bombée, concave ou convexe et peut indifféremment être de forme générale ovale, ronde, rectangulaire, octogonale, polygonale, etc.

Dans l'exemple décrit, les premiers aimants 44 sont destinés à être logés à l'intérieur du boîtier 24 des modules 14 et sont agencés pour présenter une face magnétique accessible de l'extérieur comme illustré sur **la** **figure 4****.**

On a illustré en détail sur **la** **figure 7** une partie du boîtier 24. Ce boîtier 24 comprend une coque 50 munie d'ouvertures 52. En regard de chaque ouverture 22 de la coque 50 est ménagé un logement 54 apte à recevoir chacun des éléments individuels 44. Le logement 54 est agencé de manière à ce que les aimants 44 puissent être accessibles de l'intérieur et de l'extérieur de la coque 50 à travers les ouvertures 52. Ceci permet de réaliser un raccordement électrique à la fois à l'intérieur du boîtier 24 avec notamment la source de lumière 32 mais également à l'extérieur du boîtier 24 avec un autre module 14.

Le logement 54 est formé par une encoche ouverte définie par deux rails de guidage 56 de l'aimant 44 à l'intérieur de l'encoche.

Dans l'exemple décrit, le boîtier 24 comprend en outre un couvercle 60 portant la fenêtre d'émission 26 comme cela est illustré sur **la** **figure 5****.** De préférence, lors de la fermeture du boîtier 24 par assemblage de la coque 50 avec le couvercle 60, les éléments 44 sont maintenus mécaniquement dans leur logement 54 par le couvercle 60. Par conséquent, ces éléments individuels 44 ne peuvent être séparés du module 14 une fois le boîtier 24 fermé.

Par ailleurs, le module lumineux 14 comprend des contacteurs électriques 58 aptes à créer une connexion électrique entre le circuit imprimé porté par le support 34 et les moyens de liaison 38A, 38B. Dans l'exemple illustré, les contacteurs 54 sont formés par des plots portés par le support 34. Ces plots comprennent par exemple une broche métallique apte à se déformer contre les moyens de liaisons 38A, 38B lors de l'introduction du support 34 dans le boîtier 24.

En outre, selon un mode de réalisation préféré de l'invention, l'un des moyens de liaison 38A, 38B et des moyens de liaison complémentaires 38B, 38A comprennent un élément 46 de liaison magnétique dit « mutuel » de forme générale sphérique. De préférence, cet élément mutuel sphérique 46 est entièrement séparable des deux modules 14. De préférence, cet élément mutuel sphérique 46 est formé par un deuxième aimant.

Cet élément de liaison 46 est dit « mutuel » car il peut indifféremment se fixer sur l'un ou l'autre module par attraction magnétique sur l'élément individuel 44 de l'un ou l'autre des modules. Cette désignation « mutuel » est à opposer à la désignation « individuel ».

Comme illustré en détail par **les** **figures 8A, 8B****,** de préférence, l'élément individuel 44 est apte à coopérer avec l'élément mutuel 46 selon une liaison mécanique de type sphère-plan. Bien entendu, dans des variantes non illustrées sur les figures, les moyens de liaison et les moyens de liaison complémentaires peuvent coopérer selon une liaison mécanique sphère-sphère ou sphère-creux. Dans ce cas par exemple, l'élément individuel 44 peut comporter une face creuse aimantée apte à recevoir l'élément mutuel sphérique 46 ou encore une face bombée apte à coopérer avec l'élément mutuel sphérique 46 selon une liaison sphère-sphère.

Dans le mode de réalisation préféré de l'invention, selon **la** **figure 2****,** les moyens de liaison 38A, 38B définissent un axe X passant par la paire d'éléments de liaison 40, 42. La liaison mécanique formée entre les deux modules forme un pivot le long de cet axe X. Une telle liaison permet une flexibilité des modules entre eux par articulation des modules 14 entre eux.

De préférence, afin de réaliser cette fonction de sécurité, le module lumineux comprend un capteur tactile et une surface tactile liée au capteur (non représentés sur les figures). De préférence, la surface tactile s'étend au moins partiellement sur la fenêtre d'émission.

Un capteur tactile, dit également capacitif, permet notamment de détecter si une personne touche ou effleure un objet. Un tel capteur comporte une ou plusieurs électrodes reliées à un circuit de détection de la capacitance de la ou des électrodes. Lorsqu'une personne touche ou effleure l'électrode, la capacitance de l'électrode charge et se trouve détectée par le circuit de détection qui est apte à délivrer un signal de sortie représentant l'information.

L'équipement 10 selon l'invention comprend également un module d'alimentation électrique 16.

Selon l'invention, le module d'alimentation 16 comprend un boîtier propre 24 à être saisi par un utilisateur. Ce boîtier 24 a une forme générale identique à celle du boîtier 24 des modules lumineux 14.

Par ailleurs, le module d'alimentation 16 comprend une source d'alimentation électrique 62 raccordée électriquement à des bornes électriques accessibles de l'extérieur du boîtier 24. Plus précisément, selon l'invention, les bornes électriques forment des moyens de liaison complémentaires 38A, 38B des moyens de liaison 38B, 38A du module lumineux 14. Enfin, les moyens de liaison complémentaires sont agencés pour permettre une liaison électriquement conductrice mécanique libérable entre le module d'alimentation 16 et l'un au moins des modules lumineux 14.

Ainsi, comme cela est illustré sur **les** **figures 1 et 2****,** le module d'alimentation 16 et le module lumineux 14 sont aptes à créer une liaison magnétique amovible de façon identique à la liaison magnétique amovible créée entre deux modules lumineux 14 telle que décrite ci-dessus. On ne décrira par conséquent pas plus les moyens de liaison 38A, 38B du module d'alimentation électrique 16 qui sont analogues à ceux déjà décrits en rapport aux modules lumineux 14.

La source d'alimentation électrique 62 est par exemple une batterie portative. Afin de recharger la batterie, le module d'alimentation 16 comprend une prise électrique. Par ailleurs, dans l'exemple, le module d'alimentation 16 comprend des circuits électriques diverses pour connecter la batterie aux bornes électriques d'alimentation.

On a illustré sur **les** **figures 5 et 6** une vue éclatée du module d'alimentation 16 selon ce premier mode de réalisation. On voit notamment que ce module d'alimentation 16 comprend quatre aimants 44 formant les éléments individuels agencés dans la coque 50 du boîtier 24.

Pour fabriquer le module lumineux 14 ou le module d'alimentation 16 selon le premier mode, on procède à un certain nombre d'étapes identiques. En effet, le boîtier des modules 14 et 16 est formé par assemblage d'une même coque 50 et d'un même couvercle 60 délimitant entre eux un volume intérieur fermé.

Initialement, on fait une première étape d'agencement des moyens de liaison 38A, 38B à l'intérieur de la coque 50 de manière à ce qu'ils puissent être accessibles de l'intérieur et de l'extérieur de la coque 50 à travers les ouvertures 52 ménagées dans la coque 50.

On agence l'organe principal du module 14 ou 16 à savoir, la source de lumière 32 pour le module lumineux 14 et la batterie 62 pour le module d'alimentation 16.

Dans le mode de réalisation de l'invention, lors de la fabrication du module lumineux 14, on introduit en force le support 34 dans la coque 50 afin d'établir un contact électrique des contacteurs avec les moyens de liaison 38A, 38B par déformation des broches métalliques contre les moyens de liaison 38A, 38B.

Lors de la fabrication du module d'alimentation 16, on agence la batterie 62 à l'intérieur de la coque 50 ainsi que des languettes électriquement conductrices 64 traversant la coque dans une direction transversale pour connecter électriquement les premiers 38A et seconds 38B moyens de liaison entre eux et former les bornes d'alimentation électrique du module 16.

Au cours d'une dernière étape commune à la fabrication des deux modules 14 et 16, on ferme le boîtier 24 par encliquetage de la coque 50 et respectivement du couvercle 60 comprenant la fenêtre 26 et du couvercle 61 de préférence sans fenêtre.

Dans ce premier mode de réalisation correspondant à la première utilisation illustrée par **les** **figures 1,2** **et** **12****,** les modules 14,16 sont assemblés latéralement et sont portés par le support côte à côte. Ceci permet ainsi de couvrir une surface de traitement de dimensions importantes.

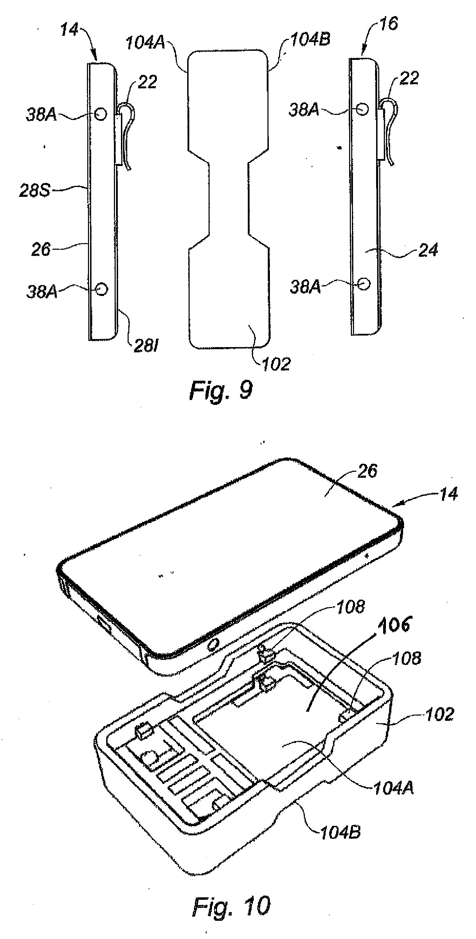

On décrira un deuxième mode en référence **aux** **figures 9 à 11** correspondant à une deuxième utilisation. Sur **ces** **figures 9 à 11****,** les éléments identiques au premier mode de réalisation portent des références identiques.

L'équipement selon ce deuxième mode de réalisation est désigné par la référence générale 100.

L'équipement 100 comprend un module d'alimentation électrique 16 et un module de traitement 14. En outre, l'équipement 100 comprend une structure 102 d'interconnexion libérable des deux modules 14 et 16 entre eux. Cette structure 102 et les modules forment un ensemble solidaire par superposition des deux modules 14 et 16 l'un sur l'autre et interconnexion des modules avec la structure. Ainsi, comme cela est illustré sur **la** **figure 9****,** le module 14 a sa face arrière 28I en regard d'une des faces du module d'alimentation 16.

Cette structure 102 est apte à relier électriquement les moyens de liaison 38A et les moyens de liaison complémentaires 38B des deux modules 14 et 16 pour autoriser le passage d'un courant électrique entre les deux modules.

Dans l'exemple illustré, la structure 102 comprend un corps 104 muni d'une cloison 106 délimitant des premier 104A et deuxième 104B espaces de réception respectivement du module lumineux 14 et du module d'alimentation 16 et des éléments 108 de contact électrique disposés selon une direction sensiblement transverse aptes à coopérer avec les moyens de liaison 38A, 38B des modules 14 et 16. De préférence, les premier 104A et deuxième 104B espaces ont une forme générale de coque.

Chaque coque 104A, 104B présente sur sa paroi interne 110 lorsque le module lumineux 14 est logé à l'intérieur les éléments de contacts 108 (non visibles) raccordées électriquement à la source d'alimentation électrique. Ces bornes sont agencées dans la coque 104A, 104B de manière à ce que lors de l'insertion du module lumineux 14 ou du module alimentation 16 à l'intérieur de la coque 104A, 104B, les bornes électriques viennent en contact électrique avec les moyens de liaison du module lumineux 14 ou les moyens de liaison du module alimentation 16.

Dans cette configuration particulière d'utilisation, on notera que les moyens de liaison 38A, 38B du module lumineux 14 et du module alimentation 16 comprennent uniquement les éléments individuels magnétiques 44. Les éléments mutuels 46 ont été séparés du module lumineux 14 et du module d'alimentation pour permettre l'insertion des modules 14 et 16 dans la structure d'interconnexion 102.

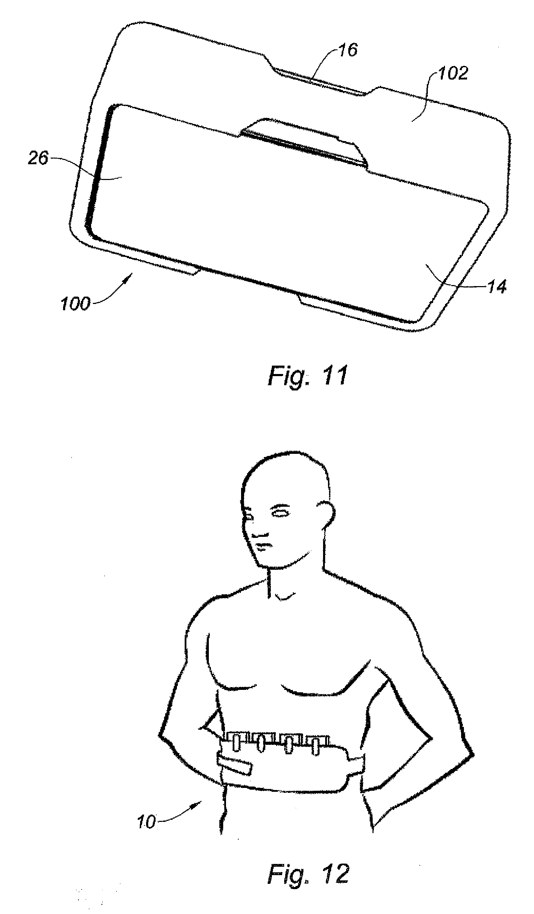

On va maintenant décrire les principaux aspects de fonctionnement de l'équipement de photothérapie 10 selon l'invention correspondant à la première configuration d'utilisation. La mise en oeuvre de l'équipement tel qu'illustré sur **la** **figure 12** se limite donc à l'assemblage des différents modules par aimantation comme cela va être détaillé ci-après.

L'utilisateur souhaite par exemple traiter une zone ventrale de son corps comme illustré sur **la** **figure 12****.** Il assemble alors plusieurs modules lumineux en mettant en contact les différents modules entre eux par liaison magnétique. Puis il assemble le module d'alimentation électrique pour former une chaîne avec les modules lumineux. Pour attacher les modules à la bande, il met à profit notamment les pinces 22.

L'utilisateur appuie alors sur le bouton marche/arrêt pour permettre l'alimentation des modules lumineux 14 par le module d'alimentation 16. Ceux-ci sont alors activés et émettent de la lumière sur la zone à traiter. On notera en outre que du fait que chaque module 14 comprend un organe de sécurité, dans le cas où l'utilisateur enlève par inadvertance un des modules lumineux 14, ce dernier n'est plus en contact avec la peau et la source lumineuse de ce module 14 va s'éteindre instantanément.

Enfin, une fois la séance de traitement terminée, l'utilisateur sépare les différents modules 14 de la chaîne par une simple force de cisaillement dans la direction perpendiculaire à l'axe principal d'aimantation. Cette force de cisaillement est réalisée selon un axe perpendiculaire à un axe principal d'aimantation de la liaison. L'équipement peut alors avantageusement être rangé dans un espace réduit.

On va maintenant décrire les principaux aspects du fonctionnement du dispositif 100 correspondant à la deuxième configuration d'utilisation. Cette configuration d'utilisation correspond à une utilisation dans laquelle l'utilisateur souhaite traiter une surface de dimension réduite, par exemple, une surface de peau présentant une cicatrice. Dans cette configuration, un seul module lumineux 14 est utilisé. L'utilisateur met alors en oeuvre l'équipement 100 illustré par **les** **figures 9 et 10****.**

Tout d'abord, il assemble le module lumineux 14 et le module de traitement 16 à l'intérieur de la structure 102 de l'équipement 100, dans chacune des coques 104A, 104B de réception. Pour cela, il lui suffit de retirer les éléments mutuels séparables 46 du module lumineux 14 et du module alimentation 16 grâce aux éléments transversaux avant d'insérer ces derniers à l'intérieur de la structure 102. Cette structure 102 permet une prise en main aisée grâce à sa forme générale ergonomique.

Une fois inséré, les deux modules 14 et 16 sont reliés électriquement par les éléments de contact transversaux ce qui permet l'alimentation électrique du module lumineux 14 par le module alimentation 16.

De ce qui précède, il ressort que l'invention remédie bien aux inconvénients signalés au début en les supprimant.

Bien entendu, d'autres modes de réalisation sont envisageables sans sortir du cadre de l'invention. Ainsi, diverses modifications peuvent être apportées par l'homme du métier à l'invention qui vient d'être décrite à titre d'exemple.

## Revendications

1. Module (14) portatif de traitement d'une zone externe d'un utilisateur par photothérapie, du type comprenant un boîtier (24) propre à être saisi par un utilisateur muni d'une fenêtre (26) d'émission d'un flux lumineux destinée à être appliquée contre la zone externe et une source (32) de génération d'un flux lumineux à travers la fenêtre (26) pour éclairer la zone, **caractérisé en ce qu**'il comprend des moyens (38A) de liaison du module (14) aptes à coopérer avec des moyens (38B) de liaison complémentaires d'un autre module (14), les moyens de liaison (38A,38B) étant agencés pour permettre une liaison mécanique libérable des deux modules (14) entre eux et étant électriquement conducteurs pour autoriser le passage d'un courant électrique entre les deux modules (14,16), les moyens de liaison (38A) et les moyens de liaison complémentaires (38B) étant de type à aimantation pour former une liaison magnétique amovible entre les deux modules (14, 16).

2. Module de traitement (14) selon la revendication précédente, dans lequel les moyens de liaison (38A) et les moyens de liaison complémentaires (38B) sont magnétiques de polarité opposée.

3. Module de traitement (14) selon l'une quelconque des revendications précédentes, dans lequel le boîtier comprend une face frontale (28S) comprenant la fenêtre d'émission et dans lequel les moyens de liaison sont disposés latéralement sur le boîtier.

4. Module de traitement (14) selon l'une quelconque des revendications précédentes, dans lequel la liaison mécanique libérable entre les deux modules (14, 16) est une liaison latérale comprenant au moins un degré de liberté.

5. Module de traitement (14) selon l'une quelconque des revendications précédentes, dans les moyens de liaison (38A) et les moyens de liaison complémentaires (38B) comprennent chacun au moins un élément de liaison magnétique (44) dit « individuel » et l'un des moyens de liaison (38A) et des moyens de liaison complémentaires (38B) comprennent un élément de liaison magnétique (46) dit « mutuel » de forme générale sphérique, cet élément mutuel sphérique (46) étant entièrement séparable des deux modules (14, 16).

6. Module de traitement (14) selon la revendication précédente, dans lequel les moyens de liaison (38A) comprenant une paire d'éléments de liaison magnétiques « individuels » (40, 42) et les moyens de liaison complémentaires (38B) comprenant une paire d'éléments de liaison magnétiques « individuels » complémentaires (42, 40), les éléments de liaison « individuels » (40, 42) de chaque paire sont magnétiques de polarité opposée afin de définir un unique sens de montage des deux modules (14, 16) entre eux.

7. Module de traitement (14) selon l'une quelconque des revendications précédentes, dans lequel la liaison mécanique des moyens de liaison (38A) et des moyens de liaison complémentaires (38B) est de type sphère-plan, sphère-sphère ou sphère-creux.

8. Module de traitement (14) selon l'une quelconque des revendications précédentes, dans lequel les moyens de liaison (38A) définissent un axe, la liaison mécanique entre les deux modules (14, 16) forme un pivot le long de cet axe.

9. Module de traitement (14) selon l'une quelconque des revendications précédentes, dans lequel le boîtier (24) comprend des premiers (38A) et seconds (38B) moyens de liaison aptes à coopérer respectivement avec des premiers (38B) et deuxièmes (38A) moyens complémentaires respectivement d'un premier autre module (14, 16) et d'un deuxième autre module (14, 16), les premiers (38A) et seconds (38B) moyens étant agencés sur deux côtés opposés du boîtier (24) pour le raccordement latéral du module (14) avec les premier et deuxième autres modules (14, 16).

10. Module de traitement (14) selon la revendication précédente, dans lequel les premiers moyens de liaison (38A) et les seconds moyens de liaison (38B) du module (14) sont complémentaires l'un de l'autre et sont magnétiques de polarité opposée.

11. Module de traitement (14) selon l'une quelconque des revendications précédentes, dans lequel la source de lumière (32) comprend une pluralité d'unités d'éclairages (36), chaque unité d'éclairage (36) comprenant au moins une puce DEL et dans lequel les unités d'éclairage (36) d'une même source sont raccordées électriquement en série les uns aux autres et les sources de deux modules liés entre eux sont raccordées électriquement en parallèle.

12. Module de traitement (14) selon l'une quelconque des revendications précédentes, comprenant un organe de sécurité d'extinction de la source de lumière en cas d'absence de contact d'au moins une partie de la fenêtre contre un objet extérieur, par exemple l'utilisateur.

13. Equipement (10) de traitement d'un utilisateur par photothérapie **caractérisé en ce qu**'il comprend une pluralité de modules (14, 16) comprenant au moins deux modules de traitement (14) selon l'une quelconque des revendications précédentes, un module (16) d'alimentation électrique du module de traitement (14) et un support (18) apte à s'étendre autour d'une partie du corps de l'utilisateur et sur lequel les modules (14, 16) sont disposés côte à côte **et en ce que** le module d'alimentation (16) comprend un boîtier (24) propre à être saisi par un utilisateur, une source d'alimentation électrique raccordée électriquement à des bornes électriques accessibles de l'extérieur du boîtier (24), les bornes électriques étant formées par des moyens de liaison complémentaires (38B) des moyens de liaison (38A) du module de traitement (14), les moyens de liaison complémentaires (38B) étant agencés pour permettre une liaison mécanique libérable des deux modules (14, 16) entre eux.

14. Equipement (10) de traitement d'un utilisateur par photothérapie, **caractérisé en ce qu**'il comprend un module (14) de traitement selon la revendication 1 et un module (16) d'alimentation électrique du module de traitement (14), **et en ce que** le module d'alimentation comprend un boîtier (24) propre à être saisi par un utilisateur, une source d'alimentation électrique raccordée électriquement à des bornes électriques accessibles de l'extérieur du boîtier (24), les bornes électriques étant formées par des moyens de liaison complémentaires (38B) des moyens de liaison (38A) du module de traitement (14), les moyens de liaison complémentaires (38B) étant agencés pour permettre une liaison mécanique libérable des deux modules (14, 16) entre eux **et en ce qu**'il comprend une structure (102) d'interconnexion libérable des deux modules (14, 16) entre eux pour former un ensemble solidaire par superposition des deux modules (14, 16) l'un sur l'autre, cette structure (102) étant apte à relier électriquement les moyens de liaison (38A) et les moyens de liaison complémentaires (38B) des deux modules (14, 16) pour autoriser le passage d'un courant électrique.

15. Equipement (10) selon la revendication précédente, dans lequel la structure (102) comprend un corps (104) muni d'une cloison (106) délimitant des premier (104A) et deuxième (104B) espaces de réception respectivement du module de traitement (14) et du module d'alimentation (16) et des éléments (108) de contact électrique disposés selon une direction sensiblement transverse aptes à coopérer avec les moyens de liaison (38A, 38B) des modules.
